# EUROPEAN PATENT APPLICATION

(11) **EP 4 772 495 A1**
(43) Date of publication of application: **08.07.2026**
(21) Application number: 24874452.6
(22) Date of filing: 12.09.2024
(51) Int. Cl.: C07C 1/12, C07B 61/00, C07C 9/04, C10L 3/08

(54) **METHANE GENERATION SYSTEM AND METHANE GENERATION METHOD**

(30) Priority: 02.10.2023 JP 2023171506
(71) Applicant: Metawater Co., Ltd., Tokyo 101-0041 (JP)
(72) Inventor: YANASE Tetsuya, Tokyo 101-0041 (JP)
(74) Representative: FDST Patentanwälte
(86) International application number: PCT/JP2024/032780
(87) International publication number: WO 2025/074841

(57) **Abstract**

A methane generation system includes: a methane generation device configured to generate methane from a first gas containing methane and carbon dioxide generated by treatment of wastewater in a wastewater treatment facility and discharges a second gas containing the generated methane; and a control device configured to control generation of the methane by the methane generation device, wherein the control device controls generation of the methane by the methane generation device according to at least one of a concentration of the carbon dioxide in the second gas, a concentration of hydrogen in the second gas, and a concentration of carbon monoxide in the second gas, and according to an amount of the carbon dioxide contained in the first gas.

## Description

### TECHNICAL FIELD

The present disclosure relates to a methane generation system and a methane generation method.

### BACKGROUND ART

In recent years, methanation for generating methane by causing carbon dioxide to react with hydrogen has been performed (see Patent Literature 1).

### CITATION LIST

### PATENT LITERATURE

Patent Literature 1: JP 2013-095681 A

### SUMMARY OF INVENTION

### PROBLEMS TO BE SOLVED BY THE INVENTION

In the methanation as described above, for example, when carbon dioxide generated by treatment of wastewater (hereinafter, also referred to as water to be treated) in a wastewater treatment facility is used, the amount and concentration of carbon dioxide supplied to a system in which methanation is performed (hereinafter, also referred to as a methanation facility) may vary, and methane generation is not stably performed in some cases. Therefore, in the methanation as described above, for example, a method capable of stably generating methane is desired.

### MEANS FOR SOLVING THE PROBLEMS

A methane generation system includes: a methane generation device configured to generate methane from a first gas containing methane and carbon dioxide generated by treatment of wastewater in a wastewater treatment facility and discharges a second gas containing the generated methane; and a control device configured to control generation of the methane by the methane generation device, wherein the control device controls generation of the methane by the methane generation device according to at least one of a concentration of the carbon dioxide in the second gas, a concentration of hydrogen in the second gas, and a concentration of carbon monoxide in the second gas, and according to an amount of the carbon dioxide contained in the first gas.

### ADVANTAGEOUS EFFECTS OF THE INVENTION

According to a methane generation system and a methane generation method in one aspect of the present disclosure, methane can be stably generated.

### BRIEF DESCRIPTION OF DRAWINGS

FIG. 1 is a diagram for explaining a configuration of the wastewater treatment facility 1000 in the first embodiment.
FIG. 2 is a diagram for explaining a configuration of the methanation facility 100 in the first embodiment.
FIG. 3 is a diagram for explaining a configuration of the methanation facility 100 in the first embodiment.
FIG. 4 is a diagram for explaining a configuration of the methanation facility 100 in the first embodiment.
FIG. 5 is a diagram for explaining a configuration of the methanation facility 100 in the first embodiment.
FIG. 6 is a flowchart for explaining the first generation control process in the first embodiment.
FIG. 7 is a diagram for explaining a configuration of the wastewater treatment facility 2000 in the second embodiment.
FIG. 8 is a diagram for explaining a configuration of the methanation facility 200 in the second embodiment.

### DESCRIPTION OF EMBODIMENTS

Hereinafter, embodiments of the present disclosure will be described with reference to the drawings. However, such description is not to be construed in a limiting sense and does not limit the claimed subject matter. In addition, various changes, substitutions, and modifications can be made without departing from the gist and scope of the present disclosure. In addition, different embodiments can be appropriately combined with each other.

### [Wastewater treatment facility 1000 in first embodiment]

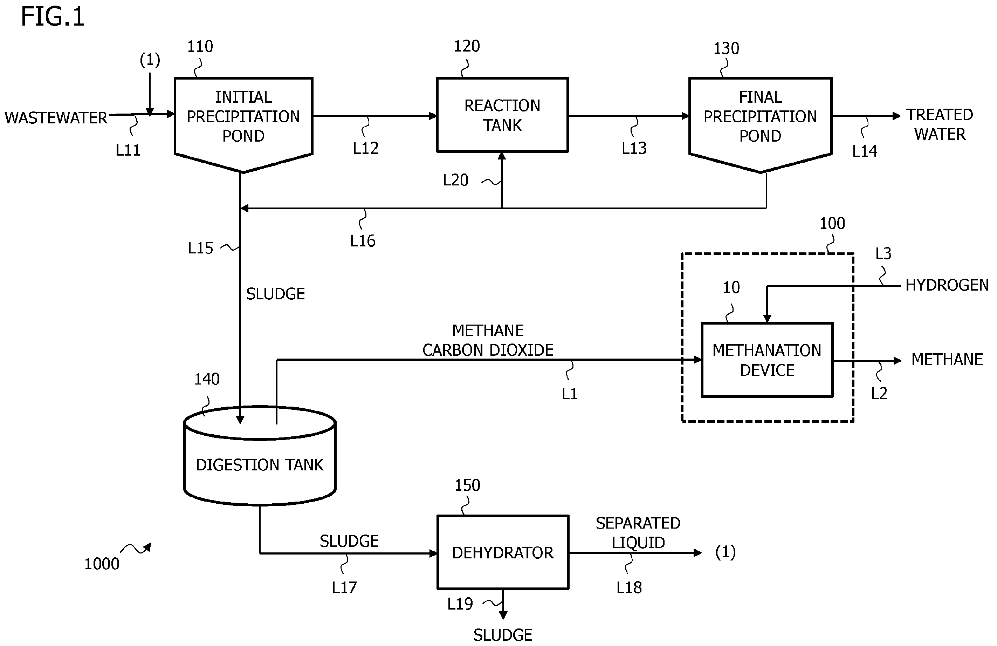

First, a wastewater treatment facility 1000 in a first embodiment will be described. FIG. 1 is a diagram for explaining a configuration of the wastewater treatment facility 1000 in the first embodiment. Note that the configuration example of each line described below is an example, and the present disclosure is not limited thereto.

As illustrated in FIG. 1, for example, the wastewater treatment facility 1000 in the present embodiment includes a methanation facility 100 (hereinafter, also referred to as a methane generation system 100), a primary sedimentation tank 110, a reaction tank 120, a final sedimentation tank 130, a digestion tank 140, and a dehydrator 150.

For example, the primary sedimentation tank 110 performs sedimentation and separation of organic matters and suspended substances (hereinafter, these are also collectively referred to as pollutants) contained in wastewater (for example, wastewater such as sewage) discharged via a line L11. The line L11 is, for example, a pipe that allows a supply source of the wastewater to communicate with the primary sedimentation tank 110. Then, for example, the primary sedimentation tank 110 discharges the separated pollutants as primary sludge from a line L15 to the digestion tank 140. The line L15 is, for example, a pipe that allows the primary sedimentation tank 110 to communicate with the digestion tank 140. In addition, for example, the primary sedimentation tank 110 discharges the wastewater obtained by separating the pollutants from a line L12 to the reaction tank 120. The line L12 is, for example, a pipe that allows the primary sedimentation tank 110 to communicate with the reaction tank 120.

Note that the line L15 may be, for example, a pipe that allows the primary sedimentation tank 110 to communicate with a concentration tank (not illustrated) and further allows the concentration tank to communicate with the digestion tank 140. In this case, for example, the concentration tank may concentrate the primary sludge discharged from the primary sedimentation tank 110 and supply the primary sludge to the digestion tank 140.

For example, the reaction tank 120 treats wastewater supplied from the primary sedimentation tank 110 via the line L12 by biological treatment (biological treatment, for example, by a standard activated sludge method or a circulating nitrification-denitrification method) with activated sludge. Then, for example, the reaction tank 120 discharges wastewater that has been subjected to the biological treatment from a line L13 to the final sedimentation tank 130. The line L13 is, for example, a pipe that allows the reaction tank 120 to communicate with the final sedimentation tank 130.

For example, the final sedimentation tank 130 performs sedimentation and separation of sludge contained in the wastewater discharged from the reaction tank 120 via the line L13. Then, for example, the final sedimentation tank 130 returns a part of the sludge that has been sedimentated and separated as return sludge from a line L16 and a line L20 to the reaction tank 120. The line L16 is, for example, a pipe that allows the final sedimentation tank 130 to communicate with the line L15. The line L20 is, for example, a pipe that allows the line L16 to communicate with the reaction tank 120. Furthermore, for example, the final sedimentation tank 130 discharges sludge other than the return sludge in the sludge that has been sedimentated and separated as excess sludge from the line L16 and the line L15 to the digestion tank 140.

Note that the line L16 may be, for example, a pipe that allows the final sedimentation tank 130 to communicate with a concentration device (not illustrated) and further allows the concentration device to communicate with the line L15. For example, the concentration device may concentrate the excess sludge discharged from the final sedimentation tank 130 and supply the excess sludge to the digestion tank 140.

In addition, for example, the final sedimentation tank 130 discharges wastewater (supernatant) obtained by separating sludge from a line L14 to a sterilization treatment device (not illustrated) at a subsequent stage. The line L14 is, for example, a pipe that allows the final sedimentation tank 130 to communicate with the sterilization treatment device. Then, for example, the sterilization treatment device (not illustrated) sterilizes the wastewater discharged from the final sedimentation tank 130 via the line L14, and discharges the sterilized treated water.

For example, the digestion tank 140 digests (decomposes) organic matters contained in each of the primary sludge supplied via the line L15 and the excess sludge supplied via the line L16 and the line L15 by anaerobic bacteria in the digestion tank 140 to generate digested sludge. Specifically, for example, the anaerobic bacteria in the digestion tank 140 generate a digested gas (hereinafter, also simply referred to as a first gas) containing methane and carbon dioxide in a process of digestion of the organic matters.

For example, the dehydrator 150 separates the digested sludge supplied from the digestion tank 140 via a line L17 into a solid matter and a separated liquid (hereinafter, also simply referred to as a separated liquid). The line L17 is, for example, a pipe that allows the digestion tank 140 to communicate with the dehydrator 150. Then, for example, the dehydrator 150 supplies the separated liquid from a line L18 to the primary sedimentation tank 110. The line L18 is, for example, a pipe that allows the dehydrator 150 to communicate with the line L11. In addition, for example, the dehydrator 150 supplies digested sludge (solid matter) obtained by separating the separated liquid from a line L19 to a subsequent stage facility (not illustrated) such as an incinerator. The line L19 is, for example, a pipe that allows the dehydrator 150 to communicate with the subsequent stage facility such as an incinerator.

As illustrated in FIG. 1, the methanation facility 100 includes, for example, a methanation device 10 (hereinafter, also referred to as a methane generation device 10) that performs methanation.

Specifically, for example, the methanation device 10 recovers the first gas generated in the digestion tank 140. Then, the methanation device 10 performs methanation, for example, by causing carbon dioxide and methane contained in the recovered first gas to react with hydrogen contained in a gas (hereinafter, also referred to as a third gas) supplied from a hydrogen supply device (not illustrated) that supplies (stores) hydrogen. The hydrogen supplied to the methanation device 10 may be, for example, hydrogen generated by electrolysis of water using renewable energy or commercially available hydrogen.

That is, for example, the methanation facility 100 in the present embodiment generates methane by using (diverting) carbon dioxide generated by treatment of wastewater in the wastewater treatment facility 1000 (digestion tank 140).

As a result, for example, the methanation facility 100 in the present embodiment can effectively use carbon dioxide generated in the wastewater treatment facility 1000. Therefore, for example, the methanation facility 100 can suppress the amount of carbon dioxide discharged from the wastewater treatment facility 1000.

### [Details of methanation facility 100 in first embodiment]

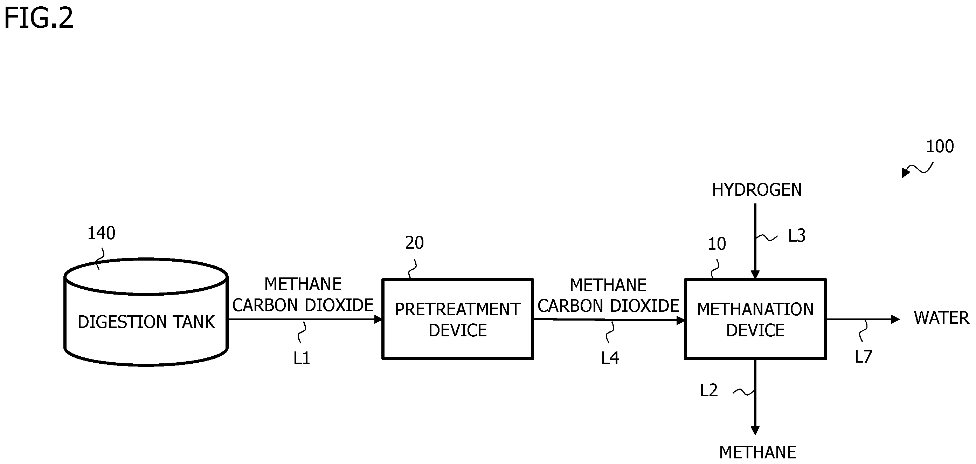

Next, the methanation facility 100 in the first embodiment will be described in detail. FIGS. 2 to 5 are diagrams for explaining a configuration of the methanation facility 100 in the first embodiment.

As illustrated in FIG. 2, the methanation facility 100 in the present embodiment includes, for example, a pretreatment device 20 in addition to the methanation device 10.

As illustrated in FIG. 2, for example, the pretreatment device 20 removes harmful substances contained in the first gas supplied from the digestion tank 140 via a line L1. Specifically, for example, the pretreatment device 20 removes sulfur compounds containing hydrogen sulfide, siloxanes, and the like from the first gas. The line L1 is, for example, a pipe that allows the digestion tank 140 to communicate with the pretreatment device 20. Then, the pretreatment device 20 supplies the first gas from which the harmful substances have been removed from a line L4 to the methanation device 10. The line L4 is, for example, a pipe that allows the pretreatment device 20 to communicate with the methanation device 10.

Note that the pretreatment device 20 may include, for example, a removal device (not illustrated) that removes hydrogen sulfide and the like contained in the first gas, and a removal device (not illustrated) that removes other sulfur compounds, siloxane, and the like contained in the first gas.

The methanation device 10 generates methane, for example, by causing carbon dioxide and methane contained in the first gas supplied from the pretreatment device 20 via the line L4 to react with hydrogen supplied from the hydrogen supply device via a line L3. The line L3 is, for example, a pipe that allows the hydrogen supply device to communicate with the methanation device 10. Then, for example, the methanation device 10 supplies a gas containing the generated methane (hereinafter, also referred to as a second gas) from a line L2 to another device such as a power generation device or a boiler (hereinafter, also simply referred to as another device). The line L2 is, for example, a pipe that allows the methanation device 10 to communicate with another device. That is, for example, the methanation device 10 supplies methane to another device that uses methane as fuel or the like. In addition, for example, the methanation device 10 supplies the second gas to an existing gas pipe (not illustrated).

Specifically, the methanation device 10 causes a reaction as indicated in the following formula 1 by using, for example, a Ni-based catalyst or a Ru-based catalyst.
[Mathematical formula 1]

CH₄ + CO₂ + H₂ → 2CH₄ + 2H₂O ... (formula 1)

As described above, for example, the methanation facility 100 in the present embodiment performs methanation using the first gas containing methane without separating methane and carbon dioxide contained in the first gas generated in the digestion tank 140.

As a result, for example, the methanation facility 100 in the present embodiment can alleviate a pace of heat generation by the presence of methane that is contained in advance and does not contribute to a catalytic reaction of methanation. Therefore, as in the above case, for example, the methanation facility 100 can suppress generation of local high temperature during a catalytic reaction of methanation, and can reduce a function of local cooling. In addition, for example, the methanation facility 100 does not need to use a device (for example, a carbon dioxide separation device or a carbon dioxide recovery device) for separating methane and carbon dioxide contained in the first gas generated in the digestion tank 140.

Therefore, for example, the methanation facility 100 can simplify the structure of the methanation device 10 and can reduce cost for generation of methane.

Note that, in the methanation performed in the methanation device 10, for example, water is generated in addition to methane as indicated in the above formula 1. Therefore, as illustrated in FIG. 2, for example, the methanation device 10 discharges water (condensed water) to the outside via a line L7. The line L7 is, for example, a pipe that allows the methanation device 10 to communicate with the outside of a building (not illustrated) in which the methanation device 10 is disposed.

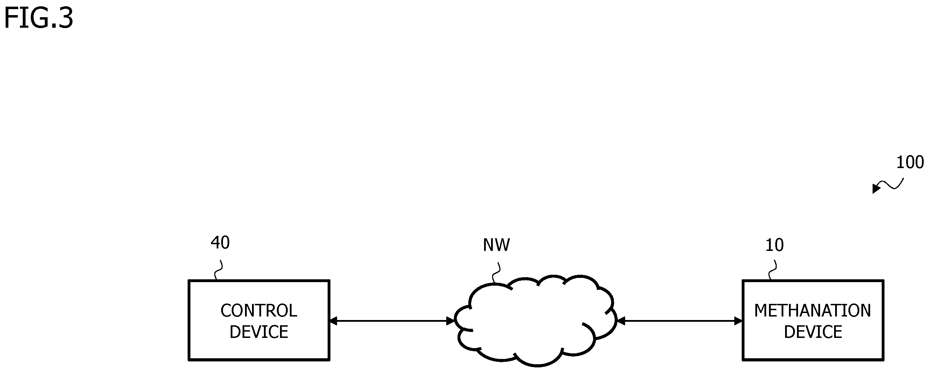

In addition, as illustrated in FIG. 3, for example, the methanation facility 100 in the present embodiment includes a control device 40 capable of communicating with the methanation device 10 via a network NW such as the Internet.

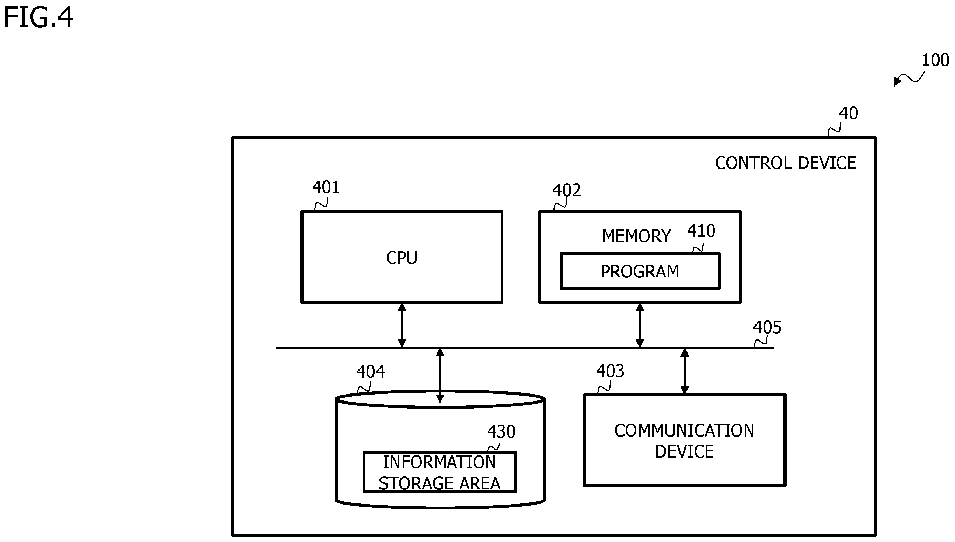

The control device 40 is, for example, an electronic device having an electronic circuit. Specifically, as illustrated in FIG. 4, the control device 40 is, for example, a computer device including a central computing unit (CPU) 401 that is a processor, a memory 402, a communication device 403, and a storage medium 404. The units are connected to each other, for example, via a bus 405. For example, the control device 40 performs a process of controlling generation of methane in the methanation device 10 (hereinafter, also referred to as a first generation control process).

For example, the storage medium 404 has a program storage area (not illustrated) that stores the program 410 for performing the first generation control process. In addition, for example, the storage medium 404 has a storage unit 430 (hereinafter, also referred to as an information storage area 430) that stores various types of information required (that may be required) for execution of the first generation control process. Specifically, the storage medium 404 may be, for example, a hard disk drive (HDD) or a solid state drive (SSD).

For example, the CPU 401 performs the first generation control process by executing the program 410 loaded from the storage medium 404 to the memory 402.

The communication device 403 communicates with, for example, the methanation device 10. In addition, the communication device 403 communicates with, for example, an operation terminal to which an administrator of the methanation facility 100 (hereinafter, also simply referred to as an administrator) inputs necessary (useful) information and the like (hereinafter, also simply referred to as an operation terminal).

Note that the electronic circuit included in the control device 40 may be, for example, a field programmable gate array (FPGA) or an application specific integrated circuit (ASIC). In this case, the first generation control process may be executed in, for example, the FPGA or the ASIC.

The control device 40 performs the first generation control process by using, for example, a value measured by each measurement device attached to the methanation device 10.

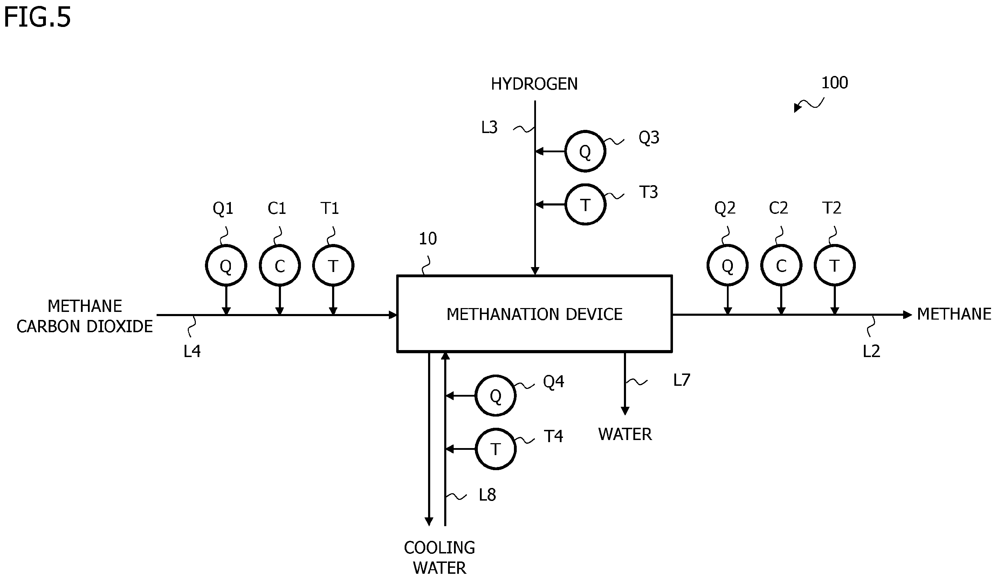

Specifically, as illustrated in FIG. 5, the control device 40 performs the first generation control process by using, for example, values measured by a flow meter Q1, a concentration meter C1, and a thermometer T1 attached to the line L4, a flow meter Q2, a concentration meter C2, and a thermometer T2 attached to the line L2, a flow meter Q3 and a thermometer T3 attached to the line L3, and a flow meter Q4 and a thermometer T4 attached to a line L8. The line L8 is, for example, a pipe (annular pipe) that allows the methanation device 10 to communicate with a cooling water circulation device (not illustrated) that circulates cooling water. The flow meter Q1, the concentration meter C1, and the thermometer T1 are, for example, measurement devices that measure a flow rate, a concentration, and a temperature of the first gas supplied from the digestion tank 140 (pretreatment device 20) to the methanation device 10, respectively. The flow meter Q2, the concentration meter C2, and the thermometer T2 are, for example, measurement devices that measure a flow rate, a concentration, and a temperature of the second gas containing methane generated in the methanation device 10, respectively. The flow meter Q3 and the thermometer T3 are, for example, measurement devices that measure a flow rate and a temperature of the third gas supplied from the hydrogen supply device to the methanation device 10, respectively. The flow meter Q4 and the thermometer T4 are, for example, measurement devices that measure a flow rate and a temperature of cooling water supplied from the cooling water circulation device to the methanation device 10, respectively.

That is, for example, the control device 40 in the present embodiment controls flow rates and the like of the first gas (a gas containing carbon dioxide and methane) and the third gas (a gas containing hydrogen) supplied to the methanation device 10 so as to suppress a concentration of a gas other than methane in the second gas generated in the methanation device 10. Specifically, for example, the control device 40 controls the flow rates and the like of the first gas and the third gas supplied to the methanation device 10 so as to suppress generation of carbon dioxide and hydrogen not used for generation of methane in the methanation device 10. In addition, for example, the control device 40 controls flow rates and the like of the first gas and cooling water supplied to the methanation device 10 so as to suppress generation of carbon monoxide caused by generation of methane in the methanation device 10.

As a result, for example, the methanation facility 100 in the present embodiment can generate methane having high purity. Therefore, for example, the methanation facility 100 can generate methane that satisfies a standard required (that may be required) in a supply destination.

Note that, for example, the concentration meter C1 may measure a concentration of carbon dioxide in the first gas. For example, the concentration meter C2 may measure at least one of a concentration of carbon dioxide, a concentration of carbon monoxide, and a concentration of hydrogen in the second gas.

For example, the control device 40 may acquire a measurement value in each of the flow meter Q1, the concentration meter C1, the thermometer T1, the flow meter Q2, the concentration meter C2, the thermometer T2, the flow meter Q3, the thermometer T3, the flow meter Q4, and the thermometer T4 (hereinafter, also collectively referred to simply as a flowmeter Q1 or the like) every predetermined time such as one minute.

### [First generation control process in first embodiment]

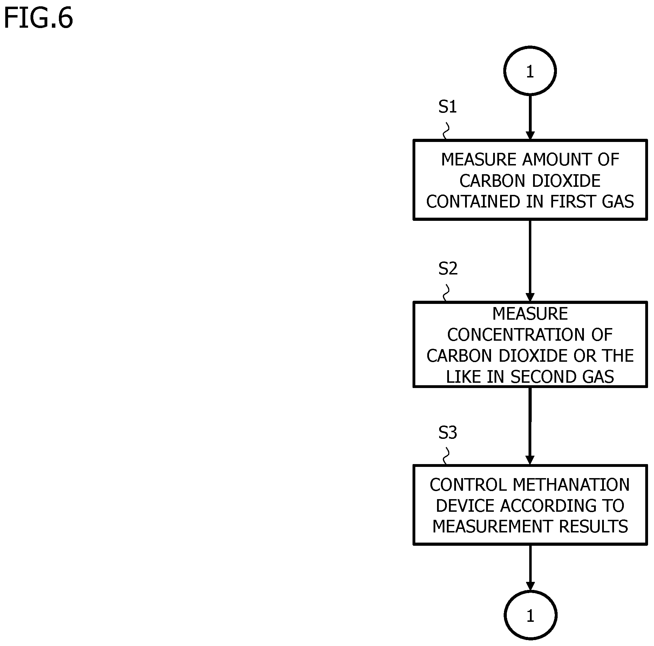

Next, the first generation control process in the first embodiment will be described. FIG. 6 is a flowchart for explaining the first generation control process in the first embodiment.

For example, the control device 40 measures a flow rate of carbon dioxide contained in the first gas supplied from the digestion tank 140 to the methanation device 10 (step S1 in FIG. 6).

Specifically, for example, the control device 40 acquires a flow rate of the first gas that is measured by the flow meter Q1 attached to the line L4, and a concentration of carbon dioxide in the first gas that is measured by the concentration meter C1 attached to the line L4. Then, for example, the control device 40 calculates a flow rate of carbon dioxide contained in the first gas by multiplying the measured flow rate of the first gas by the measured concentration of carbon dioxide in the first gas.

For example, the control device 40 measures a concentration of at least one of carbon dioxide, hydrogen, and carbon monoxide in the second gas supplied from the methanation device 10 (step S2 in FIG. 6).

Specifically, for example, the control device 40 acquires a concentration of at least one of carbon dioxide, hydrogen, and carbon monoxide in the second gas that is measured by the concentration meter C2 attached to the line L2.

Then, the control device 40 controls the methanation device 10, for example, according to the measurement results in steps S1 and S2 (step S3 in FIG. 6).

Specifically, for example, the control device 40 controls the amount of hydrogen contained in the third gas supplied from the hydrogen supply device to the methanation device 10 according to the flow rate of carbon dioxide measured in step S1.

More specifically, for example, the control device 40 calculates the amount of hydrogen (hereinafter, also referred to as a first amount) required (which may be required) when methane is generated using carbon dioxide at the flow rate measured in step S1 by following the above formula 1. Then, for example, the control device 40 performs control such that the amount of hydrogen (the amount of hydrogen contained in the third gas) supplied from the hydrogen supply device to the methanation device 10 is the first amount.

In addition, for example, the control device 40 controls at least one of the amount of carbon dioxide contained in the first gas and the amount of hydrogen contained in the third gas according to the concentration of carbon dioxide measured in step S2.

Specifically, for example, if the concentration of carbon dioxide measured in step S2 is equal to or more than a predetermined threshold (hereinafter, also referred to as a first threshold), the control device 40 performs at least one of control to decrease the amount of carbon dioxide supplied to the methanation device 10 and control to increase the amount of hydrogen supplied to the methanation device 10.

That is, for example, the above formula 1 indicates that new carbon dioxide is not generated by generation of methane. Therefore, it can be determined that the case where the concentration of carbon dioxide measured in step S2 is high is, for example, a case where a ratio of carbon dioxide not used for generation of methane in the carbon dioxide supplied to the methanation device 10 is high. In other words, it can be determined that the case where the concentration of carbon dioxide measured in step S2 is high is, for example, a case where carbon dioxide supplied to the methanation device 10 remains in the methanation device 10.

Therefore, in this case, for example, the control device 40 suppresses the amount of carbon dioxide not used for generation of methane in the methanation device 10 by reducing the amount of carbon dioxide supplied to the methanation device 10.

It can also be determined that the case where the concentration of carbon dioxide measured in step S2 is high is, for example, a case where hydrogen supplied to the methanation device 10 is insufficient in the methanation device 10.

Therefore, in this case, for example, by increasing the amount of hydrogen supplied to the methanation device 10, the control device 40 may increase the generation amount of methane in the methanation device 10 to suppress generation of carbon dioxide not used for generation of methane.

As a result, for example, the control device 40 can reduce the concentration of carbon dioxide in the second gas.

Note that, in step S2, for example, the control device 40 may acquire the concentration of carbon dioxide in the second gas that is measured by the concentration meter C2 attached to the line L2, and a flow rate of the second gas that is measured by the flow meter Q2 attached to the line L2. For example, if a product of the concentration and the flow rate measured in step S2 (that is, the amount of carbon dioxide contained in the second gas) is equal to or more than a predetermined threshold, the control device 40 may perform at least one of control to decrease the amount of carbon dioxide supplied to the methanation device 10 and control to increase the amount of hydrogen supplied to the methanation device 10.

In addition, for example, the control device 40 controls at least one of the amount of cooling water supplied to the methanation device 10 and the amount of carbon dioxide contained in the first gas according to the concentration of carbon monoxide measured in step S2.

Specifically, for example, if the concentration of carbon monoxide measured in step S2 is equal to or more than a predetermined threshold (hereinafter, also referred to as a second threshold), the control device 40 performs at least one of control to increase the amount of cooling water supplied to the methanation device 10 and control to decrease the amount of carbon dioxide supplied to the methanation device 10.

That is, the above formula 1 indicates, for example, that carbon monoxide is not generated by generation of methane. Therefore, it can be determined that the case where the concentration of carbon monoxide measured in step S2 is high is, for example, a case where a temperature in the methanation device 10 increases due to reaction heat of methane as a result of actively performed methane generation reaction in the methanation device 10, and carbon monoxide is generated together with generation of methane.

Therefore, in this case, for example, by increasing the amount of cooling water supplied to the methanation device 10, the control device 40 lowers the temperature in the methanation device 10 to suppress generation of carbon monoxide. In addition, in this case, for example, by reducing the amount of carbon dioxide supplied to the methanation device 10, the control device 40 suppresses the generation amount of methane in the methanation device 10 to lower the temperature in the methanation device 10, thereby suppressing generation of carbon monoxide.

As a result, for example, the control device 40 can reduce the concentration of carbon monoxide in the second gas.

Note that, for example, the control device 40 may measure a temperature of the second gas supplied from the methanation device 10. Specifically, for example, the control device 40 may acquire the temperature of the second gas that is measured by the thermometer T2 attached to the line L2. If the temperature of the second gas is equal to or higher than a predetermined threshold, the control device 40 may reduce the concentration of carbon monoxide in the second gas by, for example, performing at least one of control to increase the amount of cooling water supplied to the methanation device 10 and control to reduce the amount of carbon dioxide supplied to the methanation device 10.

In step S2, for example, the control device 40 may acquire a concentration of carbon monoxide in the second gas that is measured by the concentration meter C2 attached to the line L2, and a flow rate of the second gas that is measured by the flow meter Q2 attached to the line L2. For example, if a product of the concentration and the flow rate measured in step S2 (that is, the amount of carbon monoxide contained in the second gas) is equal to or more than a predetermined threshold, the control device 40 may perform at least one of control to increase the amount of cooling water supplied to the methanation device 10 and control to decrease the amount of carbon dioxide supplied to the methanation device 10.

In addition, for example, the control device 40 controls at least one of the amount of hydrogen contained in the third gas and the amount of carbon dioxide contained in the first gas according to the concentration of hydrogen measured in step S2.

Specifically, for example, if the concentration of hydrogen measured in step S2 is equal to or more than a predetermined threshold (hereinafter, also referred to as a third threshold), the control device 40 performs at least one of control to decrease the amount of hydrogen supplied to the methanation device 10 and control to increase the amount of carbon dioxide supplied to the methanation device 10.

That is, the above formula 1 indicates, for example, that hydrogen is not generated by generation of methane. Therefore, it can be determined that the case where the concentration of hydrogen measured in step S2 is high is, for example, a case where a ratio of hydrogen not used for generation of methane in the hydrogen supplied to the methanation device 10 is high. In other words, it can be determined that the case where the concentration of hydrogen measured in step S2 is high is, for example, a case where hydrogen supplied to the methanation device 10 remains in the methanation device 10.

Therefore, in this case, for example, the control device 40 suppresses the amount of hydrogen not used for generation of methane in the methanation device 10 by reducing the amount of hydrogen supplied to the methanation device 10.

It can also be determined that the case where the concentration of hydrogen measured in step S2 is high is, for example, a case where carbon dioxide supplied to the methanation device 10 is insufficient in the methanation device 10.

Therefore, in this case, for example, by increasing the amount of carbon dioxide supplied to the methanation device 10, the control device 40 may increase the generation amount of methane in the methanation device 10 to suppress generation of hydrogen not used for generation of methane.

As a result, for example, the control device 40 can reduce the concentration of hydrogen in the second gas.

Note that, in step S2, for example, the control device 40 may acquire the concentration of hydrogen in the second gas that is measured by the concentration meter C2 attached to the line L2, and a flow rate of the second gas that is measured by the flow meter Q2 attached to the line L2. For example, if a product of the concentration and the flow rate measured in step S2 (that is, the amount of hydrogen in the second gas) is equal to or more than a predetermined threshold, the control device 40 may perform at least one of control to decrease the amount of hydrogen supplied to the methanation device 10 and control to increase the amount of carbon dioxide supplied to the methanation device 10.

As described above, the methanation facility 100 in the present embodiment includes, for example, the methanation device 10 that generates methane from the first gas containing methane and carbon dioxide generated by treatment of wastewater in the wastewater treatment facility 1000 and discharges the second gas containing the generated methane. In addition, the methanation facility 100 includes, for example, the control device 40 that controls generation of methane by the methanation device 10. The control device 40 controls generation of methane by the methanation device 10 according to, for example, at least one of the concentration of carbon dioxide in the second gas, the concentration of hydrogen in the second gas, and the concentration of carbon monoxide in the second gas, and the amount of carbon dioxide contained in the first gas.

Specifically, the methanation device 10 generates methane by, for example, causing the first gas to react with the third gas containing hydrogen.

For example, the control device 40 controls the amount of hydrogen contained in the third gas according to the amount of carbon dioxide contained in the first gas.

In addition, for example, the control device 40 controls at least one of the amount of carbon dioxide contained in the first gas and the amount of hydrogen contained in the third gas according to the concentration of carbon dioxide in the second gas.

In addition, for example, the control device 40 controls at least one of the amount of cooling water supplied to the methanation device and the amount of carbon dioxide contained in the first gas according to the concentration of carbon monoxide in the second gas.

In addition, for example, the control device 40 controls at least one of the amount of hydrogen contained in the third gas and the amount of carbon dioxide contained in the first gas according to the concentration of hydrogen in the second gas.

As a result, for example, the control device 40 in the present embodiment can suppress a concentration of a gas other than methane in the second gas. Specifically, for example, even if the concentration (amount) of each gas contained in the first gas varies depending on the properties of sludge and the like and the concentration (amount) of each gas in the second gas varies as in a case where the first gas is a digested gas, the control device 40 can stably suppress the concentration of a gas other than methane in the second gas. In addition, for example, even if the concentration (amount) of each gas in the second gas varies due to a change in reaction conditions such as a temperature change of the methanation facility 100 caused by generation of methane and time degradation of a catalyst used for generation of methane, the control device 40 can stably suppress the concentration of a gas other than methane in the second gas.

As a result, for example, the methanation facility 100 in the present embodiment can stably generate methane having high purity. Therefore, for example, the methanation facility 100 can generate methane that satisfies a standard required (that may be required) in a supply destination.

Note that, in the above example, the case where the number of the methanation devices 10 included in the methanation facility 100 is one has been described, but the present disclosure is not limited thereto. Specifically, the methanation facility 100 may include, for example, a plurality of methanation devices 10 arranged in series with each other. For example, the control device 40 may control, for each of the plurality of methanation devices 10, generation of methane in each of the methanation devices 10 by using a measurement result of a gas (first gas) supplied to each of the methanation devices 10 and a measurement result of a gas (second gas) supplied from each of the methanation devices 10.

In addition, for example, the control device 40 may determine whether or not a measurement value in each of measurement devices such as the flow meter Q1 is within a normal range determined in advance for each of the measurement devices. For example, if the control device 40 detects the presence of a measurement device whose measurement value is out of the normal range, the control device 40 may output information indicating the measurement device whose presence has been detected to the operation terminal.

In addition, for example, the control device 40 may adjust the amount of carbon dioxide supplied to the methanation device 10 (the amount of carbon dioxide contained in the first gas) by adjusting an opening degree of a valve (not illustrated) disposed in a fan, a blower, or a compressor that supplies the first gas from the digestion tank 140 to the methanation device 10. In addition, for example, the control device 40 may adjust the amount of hydrogen supplied to the methanation device 10 (the amount of hydrogen contained in the third gas) by adjusting an opening degree of a valve disposed in a fan, a blower, or a compressor that supplies the third gas from the hydrogen supply device to the methanation device 10. In addition, for example, the control device 40 may adjust the amount of cooling water supplied from the cooling water circulation device to the methanation device 10 by adjusting an opening degree of a valve (not illustrated) disposed in a pump (not illustrated) that supplies cooling water from the cooling water circulation device to the methanation device 10.

In addition, for example, the control device 40 may perform control of the methanation device 10 based on measurement results in steps S1 and S2 by using a method of model predictive control that performs feedforward. Specifically, for example, the control device 40 may appropriately adjust the amount of carbon dioxide (the amount of carbon dioxide contained in the first gas) supplied to the methanation device 10 such that the concentration of carbon dioxide in the second gas discharged from the methanation device 10 is equal to or less than the first threshold.

In addition, for example, the control device 40 may perform control of the methanation device 10 based on measurement results in steps S1 and S2 by using a learning model generated by machine learning. Specifically, for example, the control device 40 may perform control using the amount of carbon dioxide output from the learning model with an input of the concentration of carbon dioxide in the second gas as the amount of carbon dioxide supplied to the methanation device 10 (the amount of carbon dioxide contained in the first gas).

In addition, for example, the control device 40 may control a pressure in the methanation device 10 such that methane is generated under a predetermined pressure.

### [Wastewater treatment facility 2000 in second embodiment]

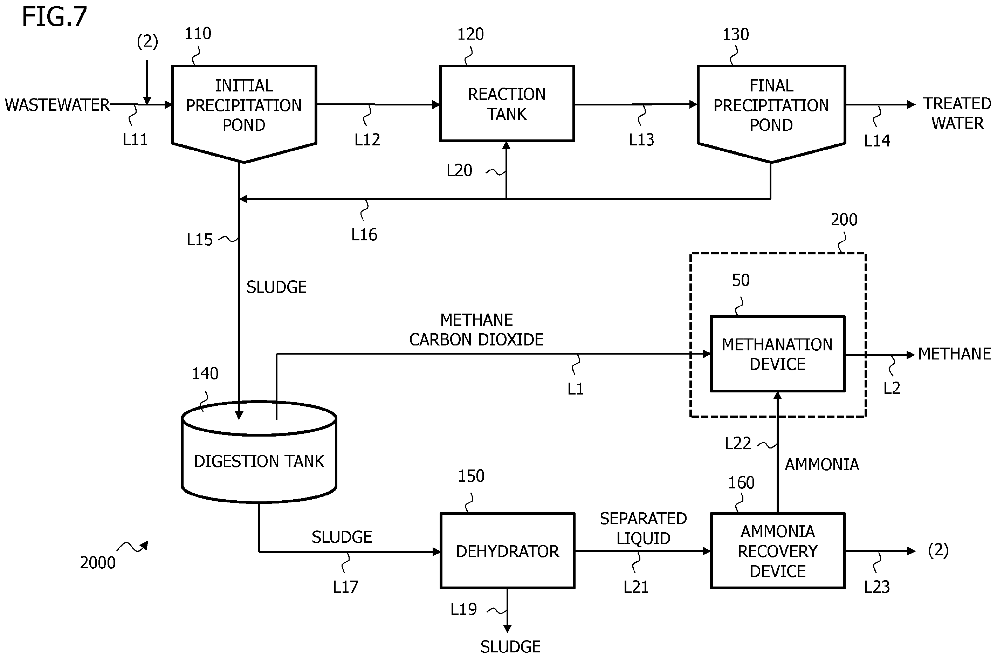

Next, a wastewater treatment facility 2000 in a second embodiment will be described. FIG. 7 is a diagram for explaining a configuration of the wastewater treatment facility 2000 in the second embodiment.

As illustrated in FIG. 7, for example, the wastewater treatment facility 2000 in the present embodiment includes a primary sedimentation tank 110, a reaction tank 120, a final sedimentation tank 130, a digestion tank 140, a dehydrator 150, an ammonia recovery device 160, and a methanation facility 200.

For example, similarly to the wastewater treatment facility 1000 in the first embodiment, the dehydrator 150 separates digested sludge supplied from the digestion tank 140 via a line L17 into a solid matter and a separated liquid. Then, for example, unlike the wastewater treatment facility 1000 in the first embodiment, the dehydrator 150 supplies the separated liquid from a line L21 to the ammonia recovery device 160. The line L21 is, for example, a pipe that allows the dehydrator 150 to communicate with the ammonia recovery device 160. In addition, for example, similarly to the wastewater treatment facility 1000 in the first embodiment, the dehydrator 150 supplies digested sludge (solid matter) obtained by separating the separated liquid from a line L19 to a subsequent stage facility (not illustrated) such as an incinerator.

For example, the ammonia recovery device 160 recovers ammonia contained in the separated liquid supplied from the dehydrator 150 via the line L21. Specifically, for example, the ammonia recovery device 160 recovers ammonia by performing stripping, zeolite adsorption/desorption, or the like. Then, for example, the ammonia recovery device 160 supplies the recovered ammonia from a line L22 to a methanation device 50 (described later). The line L22 is, for example, a pipe that allows the ammonia recovery device 160 to communicate with the methanation device 50.

In addition, for example, the ammonia recovery device 160 supplies the separated liquid after recovery of ammonia from a line L23 to the primary sedimentation tank 110. The line L23 is, for example, a pipe that directly allows the ammonia recovery device 160 to communicate with the primary sedimentation tank 110 or a pipe that allows the ammonia recovery device 160 to communicate with a line L11.

As illustrated in FIG. 7, the methanation facility 200 includes, for example, the methanation device 50 that performs methanation.

Specifically, for example, the methanation device 50 recovers a first gas (digested gas) generated in the digestion tank 140. Then, the methanation device 50 performs methanation, for example, by causing carbon dioxide and methane contained in the recovered first gas to react with ammonia contained in a gas (hereinafter, also referred to as a fourth gas) recovered in the ammonia recovery device 160.

That is, for example, similarly to the wastewater treatment facility 1000 in the first embodiment, the methanation facility 200 in the present embodiment generates methane by using (diverting) carbon dioxide generated by treatment of wastewater in the wastewater treatment facility 1000 (digestion tank 140).

As a result, for example, similarly to the wastewater treatment facility 1000 in the first embodiment, the methanation facility 200 in the present embodiment can effectively use carbon dioxide generated in the wastewater treatment facility 1000. Therefore, for example, the methanation facility 100 can suppress the amount of carbon dioxide discharged from the wastewater treatment facility 2000.

Note that, hereinafter, a case where methane is generated by using ammonia recovered in the ammonia recovery device 160 will be described, but the present disclosure is not limited thereto. Specifically, for example, the methanation facility 200 in the present embodiment may generate methane by using commercially available ammonia.

### [Details of methanation facility 200 in second embodiment]

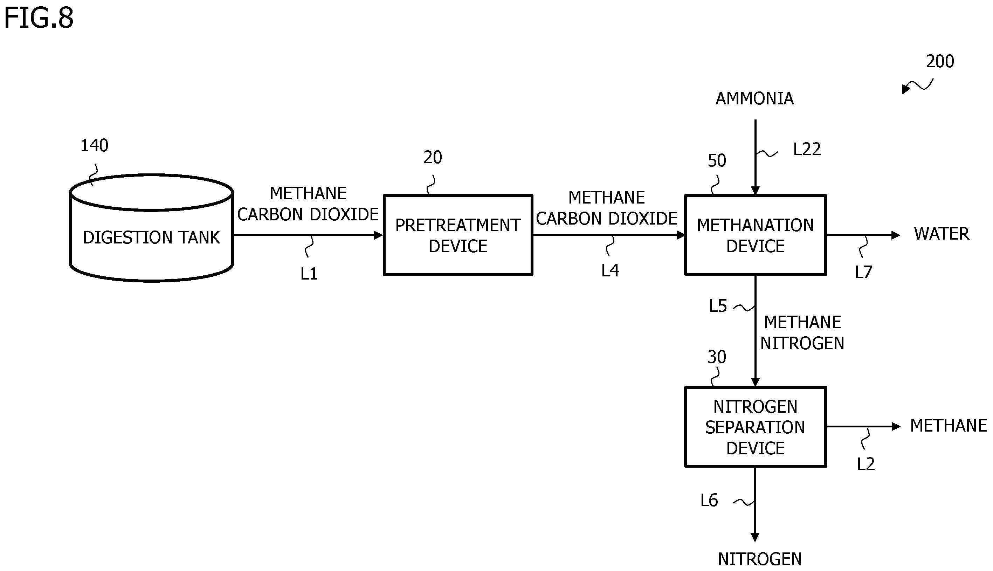

Next, the methanation facility 200 in the second embodiment will be described in detail. FIG. 8 is a diagram for explaining a configuration of the methanation facility 200 in the second embodiment.

As illustrated in FIG. 8, the methanation facility 200 in the present embodiment includes a pretreatment device 20, a nitrogen separation device 30, and the methanation device 50.

The methanation device 50 generates methane, for example, by causing carbon dioxide and methane contained in the digested gas supplied from the pretreatment device 20 via a line L4 to react with ammonia supplied from the ammonia recovery device 160 via the line L22.

Specifically, the methanation device 50 causes a reaction as indicated in the following formula 2 by using, for example, a Ni-based catalyst or a Ru-based catalyst.
[Mathematical formula 2]

CH₄ + CO₂ + 8/3NH₃ → 2CH₄ + 2H₂O + 4/3N₂... (formula 2)

Note that, in the methanation performed in the methanation device 50, for example, nitrogen and water are generated in addition to methane as indicated in the above formula 2. Therefore, as illustrated in FIG. 8, for example, the methanation device 50 discharges water (condensed water) to the outside via a line L7. In addition, as illustrated in FIG. 8, for example, the methanation device 50 supplies a gas containing methane and nitrogen to the nitrogen separation device 30 via a line L5. The line L5 is, for example, a pipe that allows the methanation device 50 to communicate with the nitrogen separation device 30.

For example, the nitrogen separation device 30 separates methane and nitrogen contained in the gas supplied from the methanation device 50 via the line L5. Then, for example, the nitrogen separation device 30 discharges nitrogen to the outside via a line L6. The line L6 is, for example, a pipe that allows the nitrogen separation device 30 to communicate with the outside of a building (not illustrated) in which the nitrogen separation device 30 is disposed. In addition, for example, the nitrogen separation device 30 supplies methane to another device such as a power generation device or a boiler via a line L2.

In addition, for example, similarly to the wastewater treatment facility 1000 in the first embodiment, the methanation facility 200 in the present embodiment includes a control device 40 capable of communicating with the methanation device 50 via a network NW such as the Internet.

For example, the control device 40 performs a process of controlling generation of methane in the methanation device 50 (hereinafter, also referred to as a second generation control process). That is, the second generation control process is, for example, a process of controlling generation of methane using ammonia.

Specifically, for example, unlike the case in the first generation control process, the control device 40 controls the amount of ammonia contained in the fourth gas according to a flow rate of carbon dioxide contained in the first gas.

More specifically, for example, the control device 40 calculates the amount of ammonia (hereinafter, also referred to as a second amount) required (which may be required) when methane is generated using carbon dioxide contained in the first gas by following the above formula 2. Then, for example, the control device 40 performs control such that the amount of ammonia (the amount of ammonia contained in the fourth gas) supplied from an ammonia supply device (not illustrated) to the methanation device 50 is the second amount.

In addition, for example, unlike the case in the first generation control process, the control device 40 controls at least one of the amount of carbon dioxide contained in the first gas and the amount of ammonia contained in the fourth gas according to the concentration of carbon dioxide in the second gas.

Specifically, for example, if the concentration of carbon dioxide in the second gas is equal to or more than a predetermined threshold, the control device 40 performs at least one of control to decrease the amount of carbon dioxide supplied to the methanation device 50 and control to increase the amount of ammonia supplied to the methanation device 50.

In addition, for example, as in the case in the first generation control process, the control device 40 controls at least one of the amount of cooling water supplied to the methanation device 50 and the amount of carbon dioxide contained in the first gas according to the concentration of carbon monoxide in the second gas.

Specifically, for example, if the concentration of carbon monoxide in the second gas is equal to or more than a predetermined threshold, the control device 40 performs at least one of control to increase the amount of cooling water supplied to the methanation device 50 and control to decrease the amount of carbon dioxide supplied to the methanation device 50.

In addition, for example, unlike the case in the first generation control process, the control device 40 controls at least one of the amount of ammonia contained in the fourth gas and the amount of carbon dioxide contained in the first gas according to the concentration of hydrogen in the second gas.

Specifically, for example, if the concentration of hydrogen in the second gas is equal to or more than a predetermined threshold, the control device 40 performs at least one of control to decrease the amount of ammonia supplied to the methanation device 50 and control to increase the amount of carbon dioxide supplied to the methanation device 50.

As described above, for example, similarly to the methanation facility 100 in the first embodiment, the methanation facility 200 in the present embodiment performs methanation using a digested gas containing methane without separating methane and carbon dioxide contained in the digested gas generated in the digestion tank 140.

As a result, for example, similarly to the methanation facility 100 in the first embodiment, the methanation facility 200 in the present embodiment can alleviate a pace of heat generation by the presence of methane that is contained in advance and does not contribute to a catalytic reaction of methanation. Therefore, for example, the methanation facility 200 can suppress generation of local high temperature during a catalytic reaction of methanation, and can reduce a function of local cooling. In addition, for example, the methanation facility 200 does not need to use a device (for example, a carbon dioxide separation device or a carbon dioxide recovery device) for separating methane and carbon dioxide contained in the digested gas generated in the digestion tank 140.

Therefore, for example, similarly to the methanation facility 100 in the first embodiment, the methanation facility 200 can simplify the structure of the methanation device 50 and can reduce cost for generation of methane.

In addition, for example, similarly to the control device 40 in the first embodiment, the control device 40 in the present embodiment can suppress a concentration of a gas other than methane in the second gas. Therefore, for example, the methanation facility 200 in the present embodiment can stably generate methane having high purity. Therefore, for example, the methanation facility 200 can generate methane that satisfies a standard required (that may be required) in a supply destination.

**REFERENCE SIGNS LIST**

| | | | |
|---|---|---|---|
| 10: | METHANATION DEVICE | | |
| 20: | PRETREATMENT DEVICE | | |
| 30: | NITROGEN SEPARATION DEVICE | | |
| 40: | CONTROL DEVICE | | |
| 50: | METHANATION DEVICE | | |
| 100: | METHANATION FACILITY | | |
| 110: | PRIMARY SEDIMENTATION TANK | | |
| 120: | REACTION TANK | | |
| 130: | FINAL SEDIMENTATION TANK | | |
| 140: | DIGESTION TANK | | |
| 150: | DEHYDRATOR | | |
| 160: | AMMONIA RECOVERY DEVICE | | |
| 200: | METHANATION FACILITY | | |
| 401: | CPU | | |
| 402: | MEMORY | | |
| 403: | COMMUNICATION DEVICE | | |
| 404: | STORAGE MEDIUM | | |
| 405: | BUS | | |
| 410: | PROGRAM | | |
| 430: | STORAGE UNIT | | |
| 1000: | WASTEWATER TREATMENT FACILITY | | |
| 2000: | WASTEWATER TREATMENT FACILITY | | |
| L1: | LINE | L2: | LINE |
| L3: | LINE | L4: | LINE |
| L5: | LINE | L6: | LINE |
| L7: | LINE | L11: | LINE |
| L12: | LINE | L13: | LINE |
| L14: | LINE | L15: | LINE |
| L16: | LINE | L17: | LINE |
| L18: | LINE | L19: | LINE |
| L20: | LINE | L21: | LINE |
| L22: | LINE | L23: | LINE |

## Claims

1. A methane generation system (100) comprising:
a methane generation device (10) configured to generate methane from a first gas containing methane and carbon dioxide generated by treatment of wastewater in a wastewater treatment facility and discharges a second gas containing the generated methane; and
a control device (40) configured to control generation of the methane by the methane generation device (10), wherein
the control device (40) controls generation of the methane by the methane generation device (10) according to at least one of a concentration of the carbon dioxide in the second gas, a concentration of hydrogen in the second gas, and a concentration of carbon monoxide in the second gas, and according to an amount of the carbon dioxide contained in the first gas.

2. The methane generation system (100) according to claim 1, wherein
the methane generation device (10) generates the methane by causing the first gas to react with a third gas containing hydrogen, and
the control device (40) controls at least one of an amount of the carbon dioxide contained in the first gas and an amount of the hydrogen contained in the third gas according to a concentration of the carbon dioxide in the second gas.

3. The methane generation system (100) according to claim 1, wherein
the methane generation device (10) generates the methane by causing the first gas to react with a third gas containing hydrogen, and
the control device (40) controls at least one of an amount of cooling water supplied to the methane generation device (10) and an amount of the carbon dioxide contained in the first gas according to a concentration of the carbon monoxide in the second gas.

4. The methane generation system (100) according to claim 1, wherein
the methane generation device (10) generates the methane by causing the first gas to react with a third gas containing hydrogen, and
the control device (40) controls at least one of an amount of the hydrogen contained in the third gas and an amount of the carbon dioxide contained in the first gas according to a concentration of the hydrogen in the second gas.

5. The methane generation system (100) according to claim 1, wherein
the methane generation device (10) generates the methane by causing the first gas to react with a fourth gas containing ammonia, and
the control device (40) controls at least one of an amount of the carbon dioxide contained in the first gas and an amount of the ammonia contained in the fourth gas according to a concentration of the carbon dioxide in the second gas.

6. A methane generation method in a methane generation system (100) comprising:
a methane generation device (10) configured to generate methane from a first gas containing methane and carbon dioxide generated by treatment of wastewater in a wastewater treatment facility and discharges a second gas containing the generated methane; and a control device (40) that controls generation of the methane by the methane generation device (10), wherein
the control device (40) controls generation of the methane by the methane generation device (10) according to at least one of a concentration of the carbon dioxide in the second gas, a concentration of hydrogen in the second gas, and a concentration of carbon monoxide in the second gas, and according to an amount of the carbon dioxide contained in the first gas.
